# EUROPEAN PATENT SPECIFICATION

(11) **EP 3 206 595 B1**
(45) Date of publication and mention of the grant of the patent: **04.12.2019**
(21) Application number: 15785193.2
(22) Date of filing: 16.10.2015
(51) Int. Cl.: A61B 17/16

(54) **CRANIAL DRILLING DEVICE**
KRANIALE BOHRVORRICHTUNG
DISPOSITIF DE FORAGE CRÂNIEN

(30) Priority: 16.10.2014 IT MO20140289
(43) Date of publication of application: 23.08.2017
(73) Proprietor: Promev S.r.l., 23900 Lecco (IT)
(72) Inventor: DALLOLIO, Villiam, 23900 Lecco (IT)
(74) Representative: Brunacci, Marco
(86) International application number: PCT/IB2015/057958
(87) International publication number: WO 2016/059608

(56) References cited:
- EP-A1- 1 038 509
- WO-A1-2011/110874
- CA-A1- 2 258 281
- DE-U1- 9 312 792
- DE-U1-202007 017 509
- US-A1- 2009 209 964

## Description

### Technical Field

The present invention relates to a cranial drilling device, usable particularly in neurosurgery.

### Background Art

All cranio-cerebral neurosurgical operations require the drilling of the skull, to allow access to the cerebral structures and the illness to be treated.

Cranial drilling tools and techniques have been known since ancient times.

In the past a manual drill was commonly used, called a "trephine", essentially comprising a substantially cylindrical blade and manually rotatable by means of a flywheel system. The hole was obtained by cutting into the skull bone, rotating the blade and applying a pressure on the skull at the same time, then removing the cylindrical core of cut bone.

Generally the trephine also comprises a guiding bit to facilitate the positioning of the tool on the point where the hole is to be made, before the blade starts to cut into the skull.

Modern trephines use specific burrs connected to common drills.

These drills have a bit with two associated burrs, particularly a first solid-bodied burr contained inside a second cylindrical burr.

Referring to the position of the drill during normal use, the two burrs are associated in order to form a substantially cylindrical lower portion, with the pointed extremity for guiding purposes, and an upper truncated-conical portion with a minimum diameter greater than the lower portion and placed near to the lower portion.

In this way the bit is able to perforate the skull bone to reach the dura mater, the outer membrane surrounding the brain.

When it no longer meets the resistance of the bone, the drill stops automatically, preventing damage to the tissues inside the skull.

These known means however have some drawbacks.

The trephine is in fact a means which became obsolete some years ago. As manual operations are required for cutting into the skull, the probability of accidental errors is very high and therefore the safety of the patient cannot be guaranteed during the operation. Moreover, the aggressiveness of the cutting profile and the lack of cutting control has led to these devices being abandoned. Concerning modern cranial drills used today, the first drawback lies in the production of non-reusable bone material.

The dual burr cuts into the bone and crushes it, producing powdered and chipped waste material that is difficult to reuse. The latter is indeed unsuitable for reuse in filling the holes made during craniotomy, given that, over time (4-6 months), phenomena of re-absorption will occur which would consequently create depressions in the skull in correspondence of the holes.

Another drawback is linked to the useful sizes of the hole made. As the extremity is of a smaller diameter compared to the upper portion, when the drill stops it forms a step, due to the fact that the upper truncated-conical portion does not penetrate through the full depth of the bone.

This step is a disadvantage for the neurosurgeon, as it actually reduces the useful diameter, creating a smaller effective diameter than that of the burr used. By way of example, when using a burr with a nominal diameter of 14 mm the useful diameter of the hole would be approximately 11 mm.

A further drawback linked to this step is given by the lengthening of surgical times associated with the risks of damaging the dura mater and/or haemorrhaging. In fact, the neurosurgeon is forced to remove this bone step using special tools such as, e.g., a laminotome, or a rosette-burr associated with the use of another type of (high-speed) drill, consequently increasing the length of the operation and the consumption of material.

Another drawback associated with the dual burr is linked to the safety of its use. In this type of device, in fact, progression into the skull only stops when the most external part comes into contact with the skull.

The progression is controlled only by the difference in length (usually between 1 and 5 mm) between the inner and outer burr, i.e. the burr moves forward automatically until it reaches a depth equal to that difference in length.

It is therefore not possible to control the progression according to the effective thickness of the skull in the area of drilling, risking the perforation of the dura mater and damage to the brain.

Document DE202007017509U U1 discloses a cranial drilling device according to the preamble of claim 1

### Disclosure of the Invention

The main aim of the present invention is to provide a cranial drilling device which allows to control the progression of the burr into the skull.

One object of the present invention is to produce a hole having a single diameter.

Another object of the present invention is to allow the portion of bone removed to be recovered for a possible re-use.

One object of the present invention is to provide a drilling device which works precisely and safely, without compromising the health and safety of the patient. Another object of the present invention is to provide a drilling device which allows to overcome the mentioned drawbacks of the prior art within the ambit of a simple, rational, easy and effective to use as well as affordable solution.

The above mentioned objects are achieved by the present cranial drilling device to use in neurosurgery, having the characteristics of claim 1.

### Brief Description of the Drawings

Other characteristics and advantages of the present invention will become better evident from the description of a preferred, but not exclusive, embodiment of a cranial drilling device, illustrated by way of an indicative, but not limitative, example in the accompanying drawings in which:
Figure 1 is an exploded view of the device according to the invention;

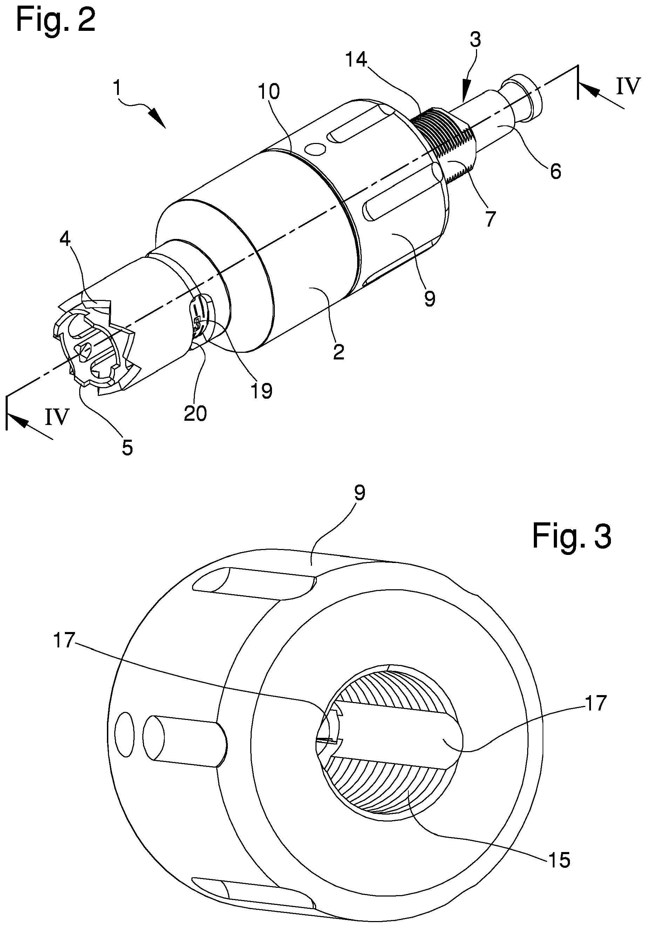

Figure 2 is an axonometric view of the drilling device according to the invention;
Figure 3 is an axonometric view of a detail of the device according to the invention;

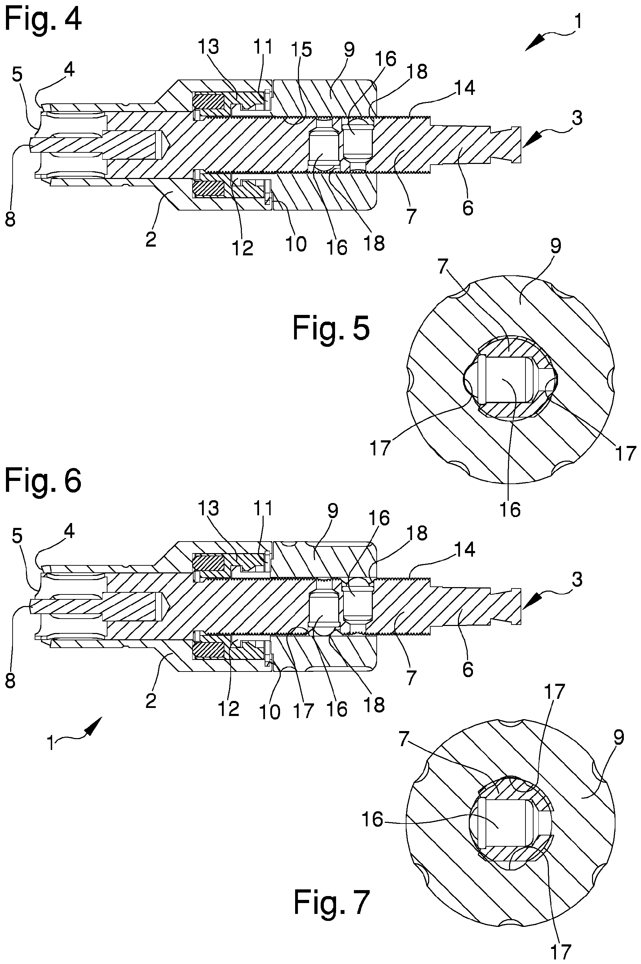

Figures 4 to 7 are sectional views which show the operation of the device according to the invention.

### Ways of carrying out the Invention

With particular reference to such figures, globally indicated by 1 is a cranial drilling device.

The cranial drilling device 1 comprises a substantially tubular stopping member 2 and a milling device 3.

The stopping member 2 has an end supporting portion 4 positionable in contact on the outer surface of a skull.

Particularly, the end supporting portion 4 comprises an indented surface able to engage onto the surface of a skull.

In the present embodiment, the indented surface is made of triangles with raw edges formed onto the end portion itself.

This conformation offers an effective grip for the indented surface on the skull bone.

The milling device 3 has a first end fitted with a hollow cutter 5 and a second end fitted with an attachment element 6 able to connect the milling device 3 to a rotating spindle.

The milling device 3 comprises a substantially elongated central member 7 placed between the first end and the second end.

In the present embodiment, the attachment element 6 is compatible with spindles commonly used in neurosurgery, but alternative solutions are not ruled out in which the attachment is assured by other or equivalent elements.

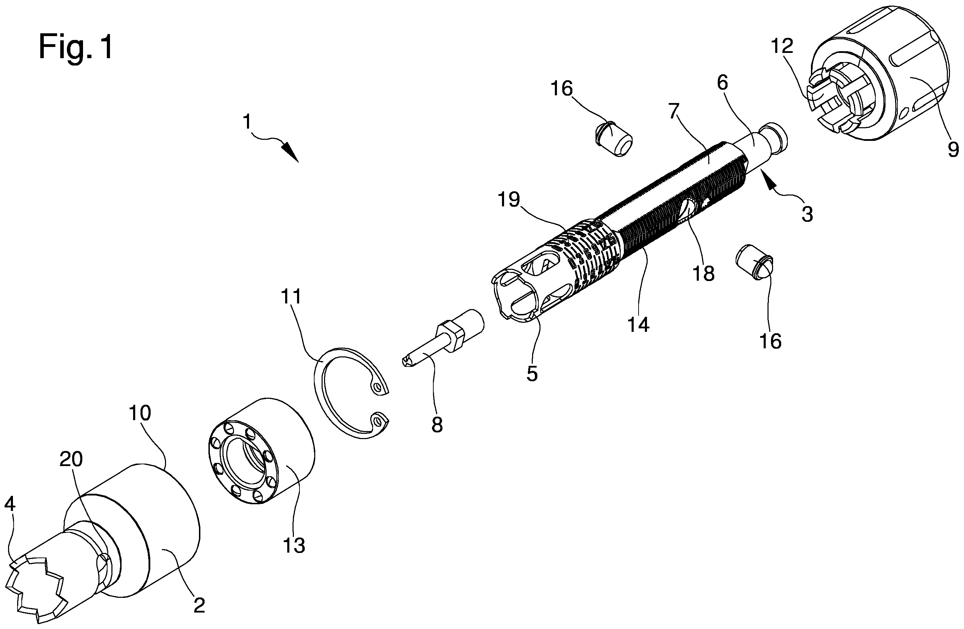

As illustrated in figure 1, the hollow cutter 5 is partly fitted in the stopping member 2 and is rotatable inside it.

According to the invention, the hollow cutter 5 is movable inside the stopping member 2 and adjusting means 9, 14, 15, 16, 17 are used to adjust the position of the hollow cutter 5 with respect to the stopping member 2.

Therefore, the hollow cutter 5 is able to slide longitudinally with respect to the stopping member 2 and the adjusting means 9, 14, 15, 16, 17 allow to adjust and therefore control the position of the hollow cutter 5 with respect to the stopping member 2.

The hollow cutter 5 has a substantially cylindrical shape and the diameter is preferably equal to 13.9 mm, i.e. the size of the cranial holes which are conventionally made in neurosurgery.

Other dimensions are not however ruled out, suited for non-standard types of operations.

In the present embodiment, the hollow cutter 5 also comprises a guiding element 8 able to facilitate the positioning of the hollow cutter 5 on the skull surface.

The guiding element 8 is inserted within the hollow cutter 5, in a substantially central position. The guiding element 8 has a double function: the main function is to centre and position the drilling device 1; the other is to create, when required, a drainage hole in the bone plug obtained.

The latter allows the outflow of biological fluids when required to control any excess cranial pressure after closing the holes.

The diameter of the guiding element 8 is variable depending on the function. When used only for centring, the diameter is preferably 0.5-1 mm.

When required for creating a drainage hole, the diameter is equal to 4-5 mm.

The adjusting means 9, 14, 15, 16, 17 comprise a ring nut element 9, sliding means 14, 15 of the ring nut element 9 along the central member 7 and locking means 16, 17 able to maintain the ring nut element 9 in a stable position with respect to the central member 7.

The ring nut element 9 is associated integral with the stopping member 2. Particularly, the top portion 10 of the stopping member 2 is in contact with the lower end of the ring nut element 9.

In the present treatise, the terms "lower" and "upper" and the like refer to the direction orthogonal to the working surface following the outwards direction from the surface.

In the present embodiment, at the interface between the ring nut element 9 and the stopping member 2 is positioned a locking ring 11, the type of a seeger ring or the like.

In the proximity of the lower end, the ring nut element 9 has a fastening element 12 able to constrain the ring nut element 9 to the stopping member 2.

This fastening element 12 is inserted through the top portion 10 of the stopping member 2 to create an interlocking and therefore making the ring nut element 9 integral with the stopping member 2.

In the present embodiment, the fastening element 12 is formed of elements protruding from the lower end of the ring nut element 9, with a hook shape which can be inserted by pressure into the top portion 10.

Alternative solutions wherein the fastening is made using different systems, such as e.g., other interlocking or similar systems are not ruled out.

The ring nut element 9 is also associated movable with the central member 7. Particularly, the ring nut element 9 fits into the central member 7 and can slide, together with the stopping member 2, along it by means of the sliding means 14, 15.

Between the stopping member 2 and the central member 7 there is advantageously a ball bearing 13 to counteract the friction developed at the interface, thus ensuring the easy rotation of the stopping member 2 with respect to the central member 7 and vice versa.

In the present embodiment the sliding means 14, 15 comprise a thread 14 made on the outer surface of the central member 7 and a counter-thread 15 made in correspondence of the inner surface of the ring nut element 9.

The thread 14 and counter-thread 15 are associated with each other so that, by rotating the ring nut element 9 around the central member 7, the ring nut element 9 can slide along the central member 7.

The locking means 16, 17 comprise at least a protruding element 16 associated with the central member 7, and at least a housing seat 17 made on the ring nut element 9.

Embodiments in which the protruding element 16 is associated with the ring nut element 9 and the housing seat 17 is made on the central member 7 are not ruled out.

The protruding element 16 is engageable in the housing seat 17 so that on each turn it clicks to control the forward movement of the ring nut element 9, and therefore of the stopping member 2, with respect to the central member 7.

In particular, the protruding element 16 is associated movable with the central member 7 along a direction substantially orthogonal to the main axis of the central member 7.

The protruding element 16 is also fitted to measure in a hole 18 made on the central member 7.

The locking means 16, 17 comprise elastic thrust means associated with the protruding element and able to push the protruding element 16 along the direction orthogonal to the axis of the central member 7.

In the present embodiment there are two protruding elements and are configured as pins with a semi-spherical exit extremity.

The protruding elements 16 are positioned in two holes 18 made in the central member 7 and directed in order to make the protruding elements 16 move outwards towards diametrically opposite points with respect to a transversal section of the central member 7.

The thrust means are contained inside the protruding elements 16 and are composed of spring bodies or the like, able to push or retract the pins according to the position of the central member 7 with respect to the ring nut element 9. The housing seat 17 comprises at least a substantially straight groove made on a portion of the inner wall of said ring nut element 9.

This groove runs longitudinally along the inner wall of the ring nut element 9. The shape of the groove is such that the protruding elements 16, when the ring nut element 9 is rotated, can be pushed and housed in the groove, stabilising the position of the ring nut element 9 with respect to the central member.

In this way the ring nut element 9 can move along the central member 7 with a step movement, where every step corresponds to a precise, controllable distance, advantageously equal to 0.50 mm.

In the present embodiment there are two housing seats 17 positioned diametrically opposite to each other, which creates a step for every 180° rotation.

Other embodiments wherein the progression of the ring nut element 9, and therefore of the stopping member 2, with respect to the central member 7 is produced in an alternative manner, e.g. using racking or toothed wheel systems or the like, are not ruled out.

Equally, other embodiments wherein the housing seat 17 is made, e.g., with a fair number of recesses or other conformations, or wherein the protruding elements 16 are made in a different manner, e.g. with fixed protrusions, or other similar solutions, are also not ruled out.

The drilling device 1 also comprises control means 19, 20 of the position of the hollow cutter 5 with respect to the stopping member 2.

Particularly, the control means 19, 20 comprise at least a numerical scale 19 made on the outer surface of the hollow cutter 5 and able to quantify the distance between a reference point of the hollow cutter 5 and a reference point of the stopping member 2.

Preferably, the reference points are the edges of the hollow cutter 5 and the edge of the end supporting portion 4.

The control means 19, 20 also comprise at least a window 20 made on the stopping member 2 and able to allow the reading of the numerical scale 19.

In this way it is possible to quantify and visually control the progression of the hollow cutter 5 in the skull. The value readable on the numerical scale at the end of drilling quantifies the final depth of progression which also represents the measurement of the thickness of the skull bone at that precise point.

In the present embodiment there are two diametrically opposite windows 20 but other solutions including more windows 20 positioned differently are not ruled out.

The operation of the present invention is the following:
By rotating the ring nut element 9 around the central member 7, the hollow cutter 5 moves forward out of the stopping member 2.

The progression depth of the hollow cutter 5 in the skull, chosen by the neurosurgeon, coincides with the distance between the edges of the hollow cutter 5 and of the end supporting portion 4. This distance is set by moving the hollow cutter forward by 0.5 mm steps with respect to the stopping member 2, i.e. by rotating the ring nut element 9 by half turn at a time until the required depth is reached.

Looking through the window 20 it is possible to control the progression of the hollow cutter 5 with respect to the stopping member 2.

After having positioned the hollow cutter 5 with respect to the stopping member 2, the drilling device is connected to the spindle and positioned on the skull surface. The guiding element 8 facilitates the correct positioning of the hollow cutter in the point where the hole 18 is to be drilled.

Rotating by the spindle, at a preferably standard speed for drills (800-1000 rpm), the hollow cutter 5 cuts into the surface of the skull and moves forward into the skull itself to a depth equal to the distance between the above-mentioned edges. At this depth, the end supporting portion 4 is in contact with the skull and grips the surrounding skull bone to prevent the hollow cutter 5 from progressing further into the skull.

If required, the neurosurgeon can further rotate the ring nut element 9 to correct the progressing depth.

After having made the hole in the skull, the hollow cutter 5 retains the cylindrical core of bone produced, which can be extracted and reused to close over the drill hole.

It has in practice been ascertained how the described invention achieves the proposed objects and particularly it is underlined how the cranial drilling device, thanks to the hollow cutter, allows to produce a hole in the skull with a single diameter and to reuse the cylindrical core of bone thus obtained.

The stepped progression and the visual control of the numerical scale also allows the neurosurgeon to drill the required depth in full safety, proceeding by subsequent steps until the cylindrical core of bone is extracted.

Thanks to the hollow cutter, the device allows to obtain a hole with the required diameter without having to subsequently intervene using other tools (laminotome, drill, etc.) to remove the steps or widen the hole already made. Moreover, again thanks to the hollow cutter, a cylindrical core of bone is obtained which can be reused subsequently to close the hole.

Thanks to the numerical scale visible on the cutter, the cranial drilling device allows to constantly read the depth and therefore the cutter's progression, and thus offers an absolute value of the skull thickness in the drilling point which is useful for assessing and taking decisions when closing.

Finally, given that the device allows a manual progression in 0.5 mm steps, always controlled by the neurosurgeon, the cutter will never penetrate into the brain, as the stopping member, in the proximity of the end supporting portion 4, will always abut the outer bone thus preventing the cutter from moving forward.

## Claims

1. Cranial drilling device (1) comprising:
- at least a substantially tubular stopping member (2) having an end supporting portion (4) positionable in contact on the surface of a skull;
- at least a milling device (3) with a first end fitted with a hollow cutter (5) and with a second end fitted with an attachment element (6) able to connect said milling device (3) to a rotating spindle and comprising a substantially elongated central member (7) placed between said first end and said second end, said hollow cutter (5) being at least partly fitted rotatable in said stopping member (2) and being movable along said stopping member (2);
- adjusting means (9, 14, 15, 16, 17) able to adjust the position of said hollow cutter (5) with respect to said stopping member (2)
wherein said adjusting means (9, 14, 15, 16, 17) comprise at least a ring nut element (9) associated integral with said stopping member (2) and associated movable with said central member (7) and sliding means (14, 15) of said ring nut element (9) along said central member (7) **characterized by** the fact that said adjusting means further comprise locking means (16, 17) able to maintain said ring nut element (9) in a stable position with respect to said central member (7), and by the fact that said locking means (16, 17) comprise at least a protruding element (16) associated with at least one of said central member (7) and said ring nut element (9), and at least a housing seat (17) made on the other of said central member (7) and said ring nut element (9), wherein said protruding element (16) is engageable in said housing seat (17).

2. Drilling device (1) according to claim 1, **characterized by** the fact that said end supporting portion (4) comprises at least an indented surface able to engage onto said surface of a skull.

3. Drilling device (1) according to one or more of the preceding claims, **characterized by** the fact that said sliding means (14, 15) comprise a thread (14) made on the outer surface of said central member (7) and a counter-thread (15) made in correspondence of the inner surface of said ring nut element (9) and associated with said thread (14).

4. Drilling device (1) according to one or more of the preceding claims, **characterized by** the fact that said protruding element (16) is associated movable with said central member (7) along a direction substantially orthogonal to the main axis of said central member (7).

5. Drilling device (1) according to one or more of the preceding claims, **characterized by** the fact that said protruding element (16) is fitted to measure in a hole (18) made on said central member (7).

6. Drilling device (1) according to one or more of the preceding claims, **characterized by** the fact that said locking means (16, 17) comprise elastic thrust means associated with said protruding element (16) and able to push said protruding element (16) along said direction substantially orthogonal to the main axis of said central member (7).

7. Drilling device (1) according to one or more of the preceding claims, **characterized by** the fact that said housing seat (17) comprises at least a substantially straight groove made on at least a portion of the inner wall of said ring nut element (9).

8. Drilling device (1) according to one or more of the preceding claims, **characterized by** the fact that it comprises control means (19, 20) of the position of said hollow cutter (5) with respect to said stopping member (2).

9. Drilling device (1) according to one or more of the preceding claims, **characterized by** the fact that said control means (19, 20) comprise at least a numerical scale (19) made on the outer surface of said hollow cutter (5) and able to quantify the distance between a reference point of said hollow cutter (5) and a reference point of said stopping member (2).

10. Drilling device (1) according to one or more of the preceding claims, **characterized by** the fact that said control means (19, 20) comprise at least a window (20) made on said stopping member (2) and able to allow the reading of said numerical scale (19).

## Patentansprüche

1. Kraniale Bohrvorrichtung (1), umfassend:
- mindestens ein im Wesentlichen rohrförmiges Anschlagelement (2) mit einem Endstützabschnitt (4), der in Kontakt auf der Oberfläche eines Schädels positionierbar ist;
- mindestens eine Fräsvorrichtung (3) mit einem ersten Ende, das mit einem Hohlfräser (5) ausgestattet ist, und mit einem zweiten Ende, das mit einem Befestigungselement (6) ausgestattet ist, das die Fräsvorrichtung (3) mit einer rotierenden Spindel verbinden kann und ein im Wesentlichen längliches Mittelelement (7) umfasst, das zwischen dem ersten Ende und dem zweiten Ende angeordnet ist, wobei der Hohlfräser (5) zumindest teilweise drehbar in dem Anschlagelement (2) angeordnet und entlang des Anschlagelements (2) beweglich ist;
- Einstellmittel (9, 14, 15, 16, 17), die die Position des Hohlfräsers (5) in Bezug auf das Anschlagelement (2) einstellen können,
wobei die Einstellmittel (9, 14, 15, 16, 17) mindestens ein Ringmutter-Element (9) umfassen, das dem Anschlagelement (2) integral zugeordnet und dem Mittelelement (7) beweglich zugeordnet ist, und Gleitmittel (14, 15) des Ringmutter-Elements (9) entlang des Mittelelements (7),
**dadurch gekennzeichnet, dass** die Einstellmittel ferner Verriegelungsmittel (16, 17) umfassen, die in der Lage sind, das Ringmutter-Element (9) in einer stabilen Position in Bezug auf das Mittelelement (7) zu halten, und dadurch, dass die Verriegelungsmittel (16, 17) mindestens ein vorstehendes Element (16), das mindestens einem von dem Mittelelement (7) und dem Ringmutter-Element (9) zugeordnet ist, und mindestens einen Gehäusesitz (17) umfassen, der auf der anderen Seite des Mittelelements (7) und des Ringmutter-Elements (9) angeordnet ist, wobei das vorstehende Element (16) in den Gehäusesitz (17) eingreifen kann.

2. Bohrvorrichtung (1) nach Anspruch 1, **dadurch gekennzeichnet, dass** der Endstützabschnitt (4) mindestens eine eingezogene Oberfläche umfasst, die in der Lage ist, auf die Oberfläche eines Schädels einzuwirken.

3. Bohrvorrichtung (1) nach einem oder mehreren der vorstehenden Ansprüche, **dadurch gekennzeichnet, dass** die Gleitmittel (14, 15) ein Gewinde (14), das auf der Außenfläche des Mittelelements (7) hergestellt ist, und ein Gegengewinde (15) umfassen, das entsprechend der Innenfläche des Ringmutter-Elements (9) hergestellt und dem Gewinde (14) zugeordnet ist.

4. Bohrvorrichtung (1) gemäß einem oder mehreren der vorhergehenden Ansprüche, **dadurch gekennzeichnet, dass** das vorstehende Element (16) dem Mittelelement (7) entlang einer Richtung, die im Wesentlichen orthogonal zu der Hauptachse des Mittelelements (7) verläuft, beweglich zugeordnet ist.

5. Bohrvorrichtung (1) nach einem oder mehreren der vorstehenden Ansprüche, **dadurch gekennzeichnet, dass** das vorstehende Element (16) maßgenau in ein Loch (18) eingepasst ist, das an dem Mittelelement (7) angebracht ist.

6. Bohrvorrichtung (1) nach einem oder mehreren der vorstehenden Ansprüche, **dadurch gekennzeichnet, dass** die Verriegelungsmittel (16, 17) elastische Schubmittel umfassen, die dem vorstehenden Element (16) zugeordnet sind und in der Lage sind, das vorstehende Element (16) entlang der Richtung zu schieben, die im Wesentlichen orthogonal zu der Hauptachse des Mittelelements (7) verläuft.

7. Bohrvorrichtung (1) nach einem oder mehreren der vorstehenden Ansprüche, **dadurch gekennzeichnet, dass** der Gehäusesitz (17) mindestens eine im Wesentlichen gerade Nut umfasst, die auf mindestens einem Abschnitt der Innenwand des Ringmutter-Elements (9) angebracht ist.

8. Bohrvorrichtung (1) nach einem oder mehreren der vorstehenden Ansprüche, **dadurch gekennzeichnet, dass** sie Steuermittel (19, 20) für die Position des Hohlfräsers (5) in Bezug auf das Anschlagelement (2) umfasst.

9. Bohrvorrichtung (1) nach einem oder mehreren der vorstehenden Ansprüche, **dadurch gekennzeichnet, dass** die Steuermittel (19, 20) mindestens eine numerische Skala (19) aufweisen, die auf der Außenfläche des Hohlfräsers (5) angebracht ist und den Abstand zwischen einem Referenzpunkt des Hohlfräsers (5) und einem Referenzpunkt des Anschlagelements (2) messen kann.

10. Bohrvorrichtung (1) nach einem oder mehreren der vorhergehenden Ansprüche, **dadurch gekennzeichnet, dass** die Steuermittel (19, 20) mindestens ein Fenster (20) umfassen, das an dem Anschlagelement (2) angebracht ist und das Lesen der numerischen Skala (19) ermöglichen kann.

## Revendications

1. Dispositif de forage crânien (1) comprenant :
- au moins un organe de butée sensiblement tubulaire (2) ayant une partie de support d'extrémité (4) positionnable en contact sur la surface d'un crâne ;
- au moins un dispositif de fraisage (3) avec une première extrémité dotée d'un outil de coupe creux (5) et avec une seconde extrémité dotée d'un élément de fixation (6) capable de relier ledit dispositif de fraisage (3) à un axe de rotation et comprenant un organe central sensiblement allongé (7) placé entre ladite première extrémité et ladite seconde extrémité, ledit outil de coupe creux (5) étant au moins partiellement inséré de manière rotative dans ledit organe de butée (2) et étant mobile le long dudit organe de butée (2) ;
- des moyens de réglage (9, 14, 15, 16, 17) capables de régler la position dudit outil de coupe creux (5) par rapport audit organe de butée (2)
dans lequel lesdits moyens de réglage (9, 14, 15, 16, 17) comprennent au moins un élément d'écrou à anneau (9) associé d'un seul tenant avec ledit organe de butée (2) et associé de manière mobile avec ledit organe central (7) et des moyens de coulissement (14, 15) dudit élément d'écrou à anneau (9) le long dudit organe central (7)
**caractérisé en ce que** lesdits moyens de réglage comprennent en outre des moyens de verrouillage (16, 17) capables de maintenir ledit élément d'écrou à anneau (9) dans une position stable par rapport audit organe central (7), et **en ce que** lesdits moyens de verrouillage (16, 17) comprennent au moins un élément faisant saillie (16) associé à au moins un dudit organe central (7) et dudit élément d'écrou à anneau (9), et au moins un siège de boîtier (17) réalisé sur l'autre dudit organe central (7) et dudit élément d'écrou à anneau (9), dans lequel ledit élément faisant saillie (16) peut venir en prise dans ledit siège de boîtier (17).

2. Dispositif de forage (1) selon la revendication 1, **caractérisé en ce que** ladite partie de support d'extrémité (4) comprend au moins une surface dentelée capable de venir en prise sur ladite surface d'un crâne.

3. Dispositif de forage (1) selon une ou plusieurs des revendications précédentes, **caractérisé en ce que** lesdits moyens de coulissement (14, 15) comprennent un filetage (14) réalisé sur la surface extérieure dudit organe central (7) et un contre-filetage (15) réalisé en correspondance de la surface intérieure dudit élément d'écrou à anneau (9) et associé audit filetage (14).

4. Dispositif de forage (1) selon une ou plusieurs des revendications précédentes, **caractérisé en ce que** ledit élément faisant saillie (16) est associé de manière mobile audit organe central (7) le long d'une direction sensiblement orthogonale à l'axe principal dudit organe central (7).

5. Dispositif de forage (1) selon une ou plusieurs des revendications précédentes, **caractérisé en ce que** ledit élément faisant saillie (16) est inséré pour mesurer dans un orifice (18) réalisé sur ledit organe central (7).

6. Dispositif de forage (1) selon une ou plusieurs des revendications précédentes, **caractérisé en ce que** lesdits moyens de verrouillage (16, 17) comprennent des moyens de poussée élastique associés audit élément faisant saillie (16) et capables de pousser ledit élément faisant saillie (16) le long de ladite direction sensiblement orthogonale à l'axe principal dudit organe central (7).

7. Dispositif de forage (1) selon une ou plusieurs des revendications précédentes, **caractérisé en ce que** ledit siège de boîtier (17) comprend au moins une rainure sensiblement rectiligne réalisée sur au moins une partie de la paroi intérieure dudit élément d'écrou à anneau (9).

8. Dispositif de forage (1) selon une ou plusieurs des revendications précédentes, **caractérisé en ce qu'**il comprend des moyens de commande (19, 20) de la position dudit outil de coupe creux (5) par rapport audit organe de butée (2).

9. Dispositif de forage (1) selon une ou plusieurs des revendications précédentes, **caractérisé en ce que** lesdits moyens de commande (19, 20) comprennent au moins une échelle numérique (19) réalisée sur la surface extérieure dudit outil de coupe creux (5) et capable de quantifier la distance entre un point de référence dudit outil de coupe creux (5) et un point de référence dudit organe de butée (2).

10. Dispositif de forage (1) selon une ou plusieurs des revendications précédentes, **caractérisé en ce que** lesdits moyens de commande (19, 20) comprennent au moins une fenêtre (20) réalisée sur ledit organe de butée (2) et capable de permettre la lecture de ladite échelle numérique (19).
